# EUROPEAN PATENT SPECIFICATION

(11) **EP 1 308 207 B1**
(45) Date of publication and mention of the grant of the patent: **10.08.2011**
(21) Application number: 02079301.4
(22) Date of filing: 16.10.2002
(51) Int. Cl.: B01J 29/06, B01J 27/045, B01J 37/20, C07C 2/58

(54) **Sulfur-containing alkylation catalyst and use thereof**
Schwefel enthaltender Alkylierungskatalysator und dessen Verwendung
Catalyseur d'alkylation contenant du soufre et son utilisation

(30) Priority: 06.11.2001 EP 01204218
(43) Date of publication of application: 07.05.2003
(73) Proprietor: Albemarle Netherlands B.V., 3818 LH Amersfoort (NL)
(72) Inventor: Broekhoven, Emanuel Hermanus, 1141 DN Monnickendam (NL); Bogaard, Pieter, 1151 EP Broek in Waterland (NL)
(74) Representative: Rasser, Jacobus Cornelis

(56) References cited:
- EP-A- 0 456 839
- EP-A- 0 640 575
- WO-A-98/23560
- GB-A- 1 117 332
- GB-A- 1 450 646
- US-A- 3 464 929
- "Webster's Online Dictionary; Definition of Trace element", , Retrieved from the Internet: URL:http://www.websters-online-dictionary. org/definitions/Trace%20element [retrieved on 2010-12-21]

## Description

The present invention pertains to a process for alkylating a hydrocarbon feed which comprises contacting the hydrocarbon feed to be alkylated with an alkylation agent in the presence of a catalyst comprising a solid acid and a hydrogenation component. The invention further relates to a catalyst suitable for said process, and to a process for the preparation of said catalyst.

Within the framework of the present invention, the term alkylation refers to the reaction of an alkylatable compound, such as an aromatic or saturated hydrocarbon, with an alkylation agent, such as an olefin. Without limiting the scope of the invention, we will further illustrate the invention by discussing the alkylation of saturated hydrocarbons, in general branched saturated hydrocarbons, with an olefin to give highly branched saturated hydrocarbons with a higher molecular weight. Hydrocarbons contain no atoms other than hydrogen and carbon.
This reaction is of interest because it makes it possible to obtain, through the alkylation of isobutane with an olefin containing 2-6 carbon atoms, an alkylate which has a high octane number and which boils in the gasoline range. Unlike gasoline obtained by cracking heavier petroleum fractions such as vacuum gas oil and atmospheric residue, gasoline obtained by alkylation is essentially free of contaminants such as sulfur and nitrogen and so has clean burning characteristics. Its high anti-knock properties, represented by the high octane number, lessen the need to add environmentally harmful anti-knock compounds such as aromatics or lead. Also, unlike gasoline obtained by reforming naphtha or by cracking heavier petroleum fractions, alkylate contains few if any aromatics or olefins, which, environmentally speaking, is a further advantage.

The alkylation reaction is acid-catalyzed. At present, in commercial alkylation equipment use is made of liquid acid catalysts such as sulfuric acid and hydrogen fluoride. The use of such catalysts is attended with a wide range of problems. For instance, sulfuric acid and hydrogen fluoride are highly corrosive, so that the equipment used has to meet high quality requirements. Since the presence of highly corrosive materials in the resulting fuel is objectionable, the remaining acid has to be removed from the alkylate. Also, because of the phase separations which have to be carried out, the process is complicated and thus expensive. Besides, there is always the risk that toxic substances such as hydrogen fluoride will be emitted.

A newer development in this field is the use of solid acid catalysts, such as zeolite-containing catalysts. WO 98/23560 discloses the use of a catalyst containing a zeolite, such as a Y zeolite, a Group VIII noble metal, e.g., platinum or palladium, as hydrogenation component, and optionally a matrix material, such as alumina, in the alkylation of hydrocarbons.

It has now been surprisingly found that if a catalyst comprising a solid acid, a hydrogenation component consisting essentially of one or more Group VIII noble metals, and at least 0.05 wt% sulfur, based on the total weight of the catalyst composition, is used in an alkylation process, the catalyst's selectivity can be considerably increased, while the activity remains essentially unchanged compared with a comparable catalyst without sulfur. This is indeed surprising, because the skilled man would expect sulfur to destroy the catalytic activity and selectivity of catalysts with a hydrogenation component consisting essentially of one or more Group VIII noble metals. See for instance J. Biswas, et al., Catal. Rev. - Sci. Eng. 30 (1988) 161-247.

The present invention is described in detail below.

### The catalyst

The catalyst used in the process is defined in the claims appended hereto and comprises a solid acid, a hydrogenation component consisting essentially of one or more Group VIII noble metals, and 0.05-1 wt% of sulfur, based on the total weight of the catalyst composition.

The solid acid is a zeolite selected from the group consisting of X-zeolites and Y-zeolites, including H-Y-zeolites and USY-zeolites. A preferred solid acid is Y-zeolite with a unit cell size of 24.34 - 24.72 angstroms.

As stated above, the hydrogenation component contained in the catalyst consists essentially of one or more Group VIII noble metals, such as platinum, palladium or a combination of platinum and palladium. Preferably, one of the Group VIII noble metals contained in the catalyst is platinum.
That the hydrogenation component contained in the catalyst consists essentially of one or more Group VIII noble metals means that hydrogenation metals different from Group VIII noble metals are essentially absent from the catalyst. Typical hydrogenation metals which thus are absent from the catalyst are any metals different from Group VIII noble metals which are able to hydrogenate unsaturated hydrocarbon compounds under the process conditions given below, such as Group VIII non-noble metals, e.g., cobalt and nickel, Group VIB metals, e.g., molybdenum and tungsten or hydrogenation metals selected from the group of rare earth metals. However, that the hydrogenation component contained in the catalyst consists essentially of Group VIII noble metals does not exclude hydrogenation metals different from Group VIII noble metals being present as impurities in small amounts, as long as they do not influence the performance of the catalyst.
The hydrogenation component is present in an amount of 0.01 to 2 wt%, preferably 0.1 to 1 wt%, calculated as metal and based on the total weight of the catalyst composition.

Apart from the solid acid and the hydrogenation component, the catalyst contains 0.05-1, preferably 0.05-0.5, and most preferably 0.1-0.25 wt% of sulfur, based on the total weight of the catalyst composition and calculated as S. These amounts do not include any sulfur, for instance in the form of sulfate or sulfonate, present in or on the solid acid prior to its combination with the hydrogenation component.
The 0.05-1 wt% sulfur can be present in various forms, for instance as elemental sulfur, as sulfate, as organic sulfur-containing compounds, or combinations thereof.
The 0.05-1 wt% sulfur can be incorporated during the contacting of the hydrogenation component with the solid acid, during a separate step which comprises contacting the material comprising both the solid acid and the hydrogenation component with a sulfur-containing compound, or a combination thereof. Preferably, sulfur is incorporated during contacting of the material comprising both the solid acid and the hydrogenation component with a sulfur-containing compound.
The 0.05-1 wt% sulfur is preferably present on and around the hydrogenation component, but can also (partly) be present on other parts of the catalyst. For instance, the 0.05-1 wt% sulfur can be present as elemental sulfur on the hydrogenation component, as sulfate on the solid acid, as organic sulfur-containing compound on the hydrogenation component, etc. Combinations thereof are also possible.

The catalyst may additionally comprise a matrix material. Examples of suitable matrix materials are alumina, silica, titania, zirconia, clays, and mixtures thereof. Matrix materials comprising alumina are generally preferred.
Preferably, the catalyst comprises 2-98 wt% of the solid acid and 98-2 wt% of the matrix material, based on the total weight of the solid acid and the matrix same as WO 98/23560 material present in the catalyst. More preferably, the catalyst comprises 10-90 wt% of the solid acid and 90-10 wt% of the matrix material, based on the total weight of the solid acid and the matrix material contained in the catalyst. Even more preferably, the catalyst comprises 10-80 wt% of the solid acid and 80-10 wt% of the matrix material, most preferably 60-90 wt% of the solid acid and 10-40 wt% of the matrix material, based on the total weight of the solid acid and the matrix material contained in the catalyst.

The catalyst contains no halogen component.

Preferably, the catalyst comprises catalyst particles wherein the ratio between (i) the volume in catalyst pores with a diameter of 40 - 8000 nm (in the following "macropores") and (ii) the specific length of the catalyst particles is in the range of 0.01 - 0.90 ml/(g*mm) and wherein the catalyst has a total pore volume of at least 0.20 ml/g.
The specific length of a catalyst particle is defined as the ratio between the geometric volume and the geometric surface of the solid part of this catalyst particle. The determination of the geometric volume and the geometric surface is known to the person skilled in the art and can be carried out, e.g., as described in DE 2354558.

The macropore volume as well as the total pore volume is determined via mercury intrusion on the basis of the Washburn equation covering the pores with a diameter of 3.6-8,000 nm.
Preferably, the ratio between the volume in macropores and the specific length is above 0.20 ml/(g*mm), more preferably above 0.30 ml/(g*mm), and even more preferably above 0.40 ml/(g*mm), as well as preferably below 0.80 ml/(g*mm).
It is further preferred that the catalyst has a total pore volume of at least 0.23 ml/g and most preferably of at least 0.25 ml/g.
The catalyst particles comprised in the catalyst preferably have a specific length of at least 0.10 mm, more preferably of at least 0.16 mm, and most preferably of at least 0.20 mm. The upper limit of the specific length preferably lies at 2.0 mm, more preferably at 1.0 mm, and most preferably at 0.6 mm.
The volume in macropores preferably is at least 0.05 ml/g, most preferably at least 0.08 ml/g, and preferably below 0.30 ml/g, most preferably below 0.25 ml/g.

The particles of the catalyst can have many different shapes, including spheres, cylinders, rings, and symmetric or asymmetric polylobes, for instance tri- and quadrulobes. Preferably, the catalyst particles have an average particle diameter of at least 0.5 mm, more preferably of at least 0.8 mm, and most preferably of at least 1.0 mm. The upper limit of the average particle diameter preferably lies at 10.0 mm, more preferably at 5.0 mm, even more preferably at 3.0 mm.

### Preparation process

The catalyst used in the alkylation process according to the invention can be prepared by contacting a material comprising the solid acid and the hydrogenation component with a sulfur-containing compound.

The material comprising the solid acid and the hydrogenation component can be prepared by processes known in the art. A typical process comprises the successive steps of
(i) shaping, e.g., extruding the solid acid, optionally after mixing it with a matrix material, to form particles,
(ii) calcining the resulting particles, and
(iii) incorporating the hydrogenation component into the calcined particles by, e.g., impregnating the particles with a solution of one or more Group VIII noble metals and/or by (competitive) ion exchange.
Alternatively, the material comprising the solid acid and the hydrogenation component can, e.g., be prepared by a process comprising the successive steps of
(i) incorporating the hydrogenation component into the solid acid or into a mixture of the solid acid and the matrix material, and
(ii) shaping, e.g., extruding the resulting material to form particles.

Preferably, the material comprising the solid acid and the hydrogenation component is calcined and subsequently reduced prior to being contacted with the sulfur-containing compound. In the above former process embodiment, these steps can be performed as steps (iv) and (v). In the above latter process embodiment, these steps can be performed as steps (iii) and (iv).

As stated above, subsequent to the preparation of the material comprising the solid acid and the hydrogenation component, the material is sulfided by being contacted with a sulfur-containing compound. Typical sulfur-containing compounds are H₂S, COS, mercaptans (e.g. CH₃SH), sulfides, disulfides (e.g., dimethylsulfide and dimethyldisulfide), and organic polysulfides, generally represented as R₁(S)ₙR₂.
The material can be contacted with the sulfur-containing compound in various ways. For example, the sulfur-containing compound is mixed or dissolved in a hydrocarbon and the resulting solution is contacted with the catalyst to be sulfided. Suitable hydrocarbons are, for instance, paraffins such as isobutane.
This contacting can be performed either before use of the resulting catalyst in the alkylation process or during the alkylation process. In the latter case, the sulfur-containing compound is added to the hydrocarbon feed to be alkylated. The so-formed hydrocarbon feed comprising a sulfur-containing compound can then be used during the entire alkylation process or only in the first part of the process, e.g., up to the first mild regeneration.
So, the present invention both relates to (i) a process for the alkylation of a hydrocarbon feed in the presence of the catalyst which has been contacted with a sulfur-containing compound before use and (ii) a process for the alkylation of hydrocarbons with a catalyst comprising a solid acid and a hydrogenation component consisting essentially of one or more Group VIII noble metals, wherein the hydrocarbon feed comprises a sulfur-containing compound.

If the catalyst has been contacted with the sulfur-containing compound before use, a subsequent activation step may be required, for instance a reduction step. Such a reduction step can be performed with hydrogen.

### The alkylation process

Preferably, the hydrocarbon to be alkylated in the alkylation process is a branched saturated hydrocarbon such as an isoalkane having 4-10 carbon atoms. Examples are isobutane, isopentane, isohexane or mixtures thereof, with isobutane being most preferred. The alkylation agent is preferably an olefin having 2-10 carbon atoms, preferably 2-6 carbon atoms, still more preferably 3-5 carbon atoms, and most preferably 4 carbon atoms. Most preferably, the alkylation process consists of the alkylation of isobutane with butenes.

As will be evident to the skilled person, the alkylation process can take any suitable form, including fluidized bed processes, slurry processes, and fixed bed processes. The process may be carried out in a number of beds and/or reactors, each with separate addition of alkylation agent, if desirable. In such a case, the process of the invention may be carried out in each separate bed or reactor.

Suitable process conditions are known to the skilled person. Preferably, an alkylation process as disclosed in WO 98/23560 is applied. The process conditions applied in the present process are summarized in the following

**Table:**

| | Temperature range [°C] | Pressure range [bar] | Molar ratio of hydrocarbon to alkylation agent |
|---|---|---|---|
| Preferred | -40 - 250 | 1 - 100 | 5:1 - 5,000:1 |
| More preferred | 20 - 150 | 5 - 40 | 50:1 - 1,000:1 |
| Most preferred | 65 - 95 | 15 - 30 | 150:1 - 750:1 |

Optionally, in the above process the catalyst may be subjected to a high-temperature regeneration with hydrogen in the gas phase. This high-temperature regeneration is preferably carried out at a temperature of at least 150°C, more preferably at 150° - 600°C, and most preferably at 200° - 400°C. For details of this regeneration procedure, reference is made to WO 98/23560, and in particular to page 4, lines 12-19. The high-temperature regeneration can, e.g., be applied periodically during the alkylation process or, alternatively, the catalyst can be subjected to this high-temperature regeneration treatment after being contacted with the sulfur-containing compound and prior to being contacted with the hydrocarbon feed and the alkylating agent.

Preferably, in addition to the high-temperature regeneration treatment a milder regeneration is applied during the alkylation process, such as described in WO 98/23560, in particular page 9, line 13 through page 13, line 2. More in particular, during the alkylation process the catalyst is preferably subjected intermittently to a regeneration step by being contacted with a feed containing a hydrocarbon and hydrogen, with said regeneration preferably being carried out at 90% or less, more preferably at 60% or less, even more preferably at 20% or less, and most preferably at 10% or less of the active cycle of the catalyst. The active cycle of the catalyst is defined as the time from the start of the feeding of the alkylation agent to the moment when, in comparison with the alkylation agent added to the catalyst-containing reactor section, 20% of the alkylation agent leaves the catalyst-containing reactor section without being converted, not counting isomerization inside the molecule.

The quality of the alkylate product obtained in the process according to the invention can be measured by its Research Octane Number (RON). The RON is a measure of the anti-knock rating of gasoline and/or gasoline constituents. The higher the RON, the more favourable the anti-knock rating of the gasoline will be. Depending on the type of gasoline engine, generally speaking a higher anti-knock rating is of advantage when it comes to the working of the engine. The product obtained in the process according to the invention preferably has a RON of 90 or higher, more preferably of 92 or higher, most preferably 94 or higher. The RON is obtained by determining, e.g., via gas chromatography, the percentages by volume of the various hydrocarbons in the product. The percentages by volume are then multiplied by the RON contribution and added up.
Examples of compounds with a RON of 90 or higher are isopentane, 2,2-dimethyl butane, 2,3-dimethyl butane, trimethyl butane, 2,3-dimethyl pentane, 2,2,4-trimethyl pentane, 2,2,3-trimethyl pentane, 2,3,4-trimethyl pentane, 2,3,3-trimethyl pentane, and 2,2,5-trimethyl hexane.

A related relevant parameter for product quality is the ratio of the amount of formed trimethyl pentanes (TMP) to the amount of formed dimethyl hexanes (DMH). Trimethyl pentanes have a RON of about 100-110. Dimethyl hexanes have a RON of about 60-70. Consequently, to obtain an alkylate with a high octane number, the highest possible TMP/DMH ratio is desired. The process according to the invention makes it possible to obtain a product having a TMP/DMH ratio of at least 2, preferably of at least 3, more preferably of at least 4.

The alkylation process according to the invention results in a high conversion of the alkylation agent (the amount of alkylation agent in the feed that is converted in the reaction), a high C5+ alkylate yield (the weight amount of C5+ alkylate produced divided by the overall weight of alkylation agent consumed), and a good stability, while the selectivity as characterized by the RON and the TMP/DMH ratio has improved over the unsulfided catalyst.

The invention will be described by way of the examples given below.

### EXAMPLES

### General test procedure

An amount of catalyst corresponding to 5 grams of zeolite calcined at 400°C was put in a measuring cylinder of 50 ml with an internal diameter of 2.5 cm. Carborundum particles with a particle size of 16 mesh were added to a total volume of 30 ml. The catalyst and the carborundum particles were carefully mixed. In a reactor with an internal diameter of 15 mm, equipped with a thermowell with an external diameter of 3 mm in the centre of the reactor, the bottom 17 cm were filled with carborundum particles with a diameter of 16 mesh, which were covered with a thin layer of glass wool. Then, the mixture of catalyst and carborundum particles was added, followed by the large voids in the catalyst bed being filled with fine carborundum (100 mesh), while tapping against the reactor. The catalyst layer was covered with a thin layer of glass wool, and topped off with 16 mesh carborundum particles.

After being closed and purged with nitrogen, the reactor was brought under a H₂ flow of 1 Nl/min and heated to 90°C under atmospheric pressure. The reactor temperature was then raised to 200°C in about 45 minutes. After 1 hour at 200°C the temperature was raised to 400°C at a rate of 2°C/min, and the reactor was kept at this temperature for half an hour. Subsequently, the reactor temperature was allowed to fall during the night to 90°C, which was the reaction temperature. Then, the pressure was set at 21 bar, and a flow of isobutane, followed by a flow of a mixture of 2% of cis-2-butene in isobutane, was led over the catalyst.

The catalysts were tested at a temperature of 90°C, a pressure of 21 bar, a ratio of isobutane to cis-2-butene of 50:1, and an olefin weight space velocity (WHSV), calculated on total zeolite, of 0.4 h⁻¹.
Breakthrough runs were carried out, followed each time by regeneration of the catalyst with gaseous hydrogen at 250°C and 21 bar. With this procedure, successive breakthrough runs with the same catalyst sample could be performed.

A parameter indicating the stability and activity of the catalyst is the catage before olefin breakthrough. The catage is calculated as follows. Multiplying the C5+ alkylate yield, expressed as ratio, by the space velocity of the olefin gives the C5+ alkylate yield expressed in grams of C5+ alkylate per hour per gram of catalyst. Multiplying this C5+ alkylate yield by the number of hours of production gives the catage.

### Comparative Example A

A catalyst comprising a 70 wt% USY-zeolite, 0.34 wt% platinum, and 30 wt% alumina was tested according to the above-described general test procedure. The catalytic performance is given in the Table below.

### Example 1

### Experiment 1A

After regeneration with H₂ at 250°C the catalyst used in Comparative Example A was again tested according to the above-described general test procedure. However, in this case the catalyst was contacted for 200 minutes with an isobutane and olefin feed, which also contained 1,200 ppm S in the form of H₂S.

### Experiment 1B

After regeneration in hydrogen at 250°C, the catalyst used in experiment 1A was again tested according to the general test procedure for 200 minutes with a feed containing 1,200 ppm S in the form of H₂S.

### Experiment 1C

The catalyst used in Experiment 1B was again regenerated and tested according to Experiment 1B.

### Experiment 1D

The catalyst used in Experiment 1C was again regenerated and tested according to Experiment 1B.

### Experiment 1E

The catalyst used in Experiment 1D was again regenerated in hydrogen at 250°C. The catalyst was subsequently tested according to the general test procedure, i.e. with no sulfur in the isobutane feed.
The catage, RON, and TMP/DMH obtained in these experiments are given in the Table below. After the test the catalyst contained 0.25 wt% of sulfur.

### Discussion

The catalytic performance of the catalysts of the above Examples is summarized in the following Table: .

| | Catage (g C₅⁺/g catalyst) | RON | TMP/DMH |
|---|---|---|---|
| Comparative Example A | >2.00 | 89.70 | 2.4 |
| Experiment 1A | 0.91 | 90.86 | 2.8 |
| Experiment 1B | 0.93 | 90.74 | 2.7 |
| Experiment 1C | 0.97 | 90.76 | 2.7 |
| Experiment 1D | 1.04 | 91.26 | 2.9 |
| Experiment 1E | >2.00 | 91.02 | 2.8 |

From this table it is clear that the presence of sulfur in the isobutane feed (Experiments 1A-1D) has a negative influence on the catage. However, surprisingly, the selectivity of the catalyst increases with the presence of sulfur in the feed.
This table also shows that upon using a sulfided catalyst in the absence of sulfur in the isobutane feed (Experiment 1E), the catage of the catalyst returns to the level of an unsulfided catalyst (Comparative Example A), while the selectivity remains at the level of Experiments 1A-1D.

## Claims

1. A process for alkylating a saturated hydrocarbon feed which comprises contacting the hydrocarbon feed to be alkylated with an alkylation agent in the presence of a catalyst comprising:
a solid acid, wherein said solid acid is a zeolite selected from the group consisting of X-zeolites and Y-zeolites, including H-Y-zeolites and USY-zeolites;
a hydrogenation component consisting essentially of one or more Group VIII noble metals, said hydrogenation component being present in an amount of from 0.01 to 2 wt.% calculated as metal and based on the total weight of the catalyst composition,
0.05 - 1 wt% of sulfur, based on the total weight of the catalyst composition and calculated as S, and
no halogen component.

2. A process according to claim 1, wherein the hydrocarbon feed comprises an isoalkane having 4-10 carbon atoms and the alkylation agent comprises an olefin comprising 2-10 carbon atoms.

3. A process for alkylating a hydrocarbon feed which comprises contacting the saturated hydrocarbon feed to be alkylated with an alkylation agent in the presence of a catalyst comprising:
a solid acid, wherein said solid acid is a zeolite selected from the group consisting of X-zeolites and Y-zeolites, including H-Y-zeolites and USY-zeolites;
a hydrogenation component consisting essentially of one or more Group VIII noble metals, said hydrogenation component being present in an amount of from 0.01 to 2 wt.%, calculated as metal and based on the total weight of the catalyst composition,
0.05 - 1 wt.% of sulphur based on the total weight of the catalyst composition and calculated as S, and
no halogen component,
wherein the hydrocarbon feed comprises a sulfur-containing compound.

4. A process according to claim 3, wherein the solid acid comprises Y-zeolite with a unit cell size of 24.34 - 24.72 angstroms.

5. A process according to any one of claims 1 to 4, wherein the Group VIII noble metal is selected from platinum, palladium, and mixtures thereof.

6. A process according to any one of claims 1 to 5, wherein the hydrogenation component comprises platinum.

7. A catalyst suitable for use in the alkylation of hydrocarbons which comprises:
a zeolite selected from the group consisting of X-zeolites and Y-zeolites, including H-Y-zeolites and USY-zeolites;
a hydrogenation component consisting essentially of one or more Group VIII noble metals but at any rate comprising platinum, said hydrogenation component being present in an amount of from 0.01 to 2 wt.%, calculated as metal and based on the total weight of the catalyst composition,
0.05 - 1 wt% of sulfur based on the total weight of the catalyst composition and calculated as S, and
no halogen component.

8. A catalyst according to claim 7, wherein the zeolite comprises Y-zeolite with a unit cell size of 24.34 - 24.72 angstroms.

9. A process for preparing the catalyst of claims 7 **or claim 8** which process comprises the step of contacting a material comprising the zeolite and the hydrogenation component with a sulfur-containing compound.

10. A process according to claim 9, wherein the material comprising the zeolite and the hydrogenation component is calcined and subsequently reduced prior to being contacted with the sulfur-containing compound.

11. A process according to claim 9 or 10, wherein the material comprising the zeolite and the hydrogenation component is reduced after being contacted with the sulfur-containing compound.

## Patentansprüche

1. Verfahren zum Alkylieren von gesättigten Kohlenwasserstoffen, das das Kontaktieren der zu alkylierenden Kohlenwasserstoffe mit einem Alkylierungsmittel in Gegenwart eines Katalysators umfasst, umfassend:
eine feste Säure, wobei die feste Säure ein Zeolith ist, ausgewählt aus der Gruppe bestehend aus X-Zeolithen und Y- Zeolithen, einschließlich H-Y-Zeolithen und USY-Zeolithen,
eine Hydrierkomponente, die im Wesentlichen aus einem oder mehreren Edelmetallen der Gruppe VIII besteht, wobei die Hydrierkomponente in einer Menge von 0,01 bis 2 Gew.-Proz., als Metall und bezogen auf das Gesamtgewicht der Katalysatorzusammensetzung berechnet, vorhanden ist,
0,05 bis 1 Gew.-Proz. Schwefel, bezogen auf das Gesamtgewicht der Katalysatorszusammensetzung und als S berechnet, und
keine Halogenkomponente.

2. Verfahren nach Anspruch 1, wobei die Kohlenwasserstoffe ein Isoalkan mit 4-10 Kohlenstoffatomen umfassen und das Alkylierungsmittel ein Olefin mit 2-10 Kohlenstoffatomen umfasst.

3. Verfahren zum Alkylieren von gesättigten Kohlenwasserstoffen, das das Kontaktieren der zu alkylierenden Kohlenwasserstoffe mit einem Alkylierungsmittel in Gegenwart eines Katalysators umfasst, umfassend:
eine feste Säure, wobei die feste Säure ein Zeolith ist, ausgewählt aus der Gruppe bestehend aus X-Zeolithen und Y- Zeolithen, einschließlich H-Y-Zeolithen und USY-Zeolithen,
eine Hydrierkomponente, die im Wesentlichen aus einem oder mehreren Edelmetallen der Gruppe VIII besteht, wobei die Hydrierkomponente in einer Menge von 0,01 bis 2 Gew.-Proz., als Metall und bezogen auf das Gesamtgewicht der Katalysatorzusammensetzung berechnet wird, vorhanden ist,
0,05 bis 1 Gew.-Proz. Schwefel, bezogen auf das Gesamtgewicht der Katalysatorszusammensetzung und als S berechnet, und
keine Halogenkomponente,
wobei die Kohlenwasserstoffe eine schwefelhaltige Verbindung umfassen.

4. Verfahren nach Anspruch 3, wobei die feste Säure Y-Zeolith mit einer Elementarzellengröße von 24,34 bis 24,72 Angström umfasst.

5. Verfahren nach einem der Ansprüche 1 bis 4, wobei das Edelmetall der Gruppe VIII aus Platin, Palladium und Mischungen davon ausgewählt wird.

6. Verfahren nach einem der Ansprüche 1 bis 5, wobei die Hydrierkomponente Platin umfasst.

7. Katalysator, der für den Einsatz in der Alkylierung von Kohlenwasserstoffen brauchbar ist, umfassend:
ein Zeolith, ausgewählt aus der Gruppe bestehend aus X-Zeolithen und Y- Zeolithen, einschließlich H-Y-Zeolithen und USY-Zeolithen,
eine Hydrierkomponente, die im Wesentlichen aus einem oder mehreren Edelmetallen der Gruppe VIII besteht und auf jeden Fall Platin umfasst, wobei die Hydrierkomponente in einer Menge von 0,01 bis 2 Gew.-Proz., als Metall und bezogen auf das Gesamtgewicht der Katalysatorzusammensetzung berechnet, vorhanden ist,
0,05 bis 1 Gew.-Proz. Schwefel, bezogen auf das Gesamtgewicht der Katalysatorszusammensetzung und als S berechnet, und
keine Halogenkomponente.

8. Katalysator nach Anspruch 7, wobei der Zeolith Y-Zeolith mit einer Elementarzellengröße von 24,34 bis 24,72 Angström umfasst.

9. Verfahren zum Herstellen des Katalysators von Anspruch 7 oder Anspruch 8, wobei das Verfahren den Schritt des Kontaktierens eines Materials, das den Zeolith und die Hydrierkomponente umfasst, mit einer schwefelhaltigen Verbindung umfasst.

10. Verfahren nach Anspruch 9, wobei das Material, das den Zeolith und die Hydrierkomponente umfasst, calciniert und anschließend reduziert wird, bevor es mit der schwefelhaltigen Verbindung kontaktiert wird.

11. Verfahren nach Anspruch 9 oder 10, wobei das Material, das den Zeolith und die Hydrierkomponente umfasst, reduziert wird, nachdem es mit der schwefelhaltigen Verbindung kontaktiert worden ist.

## Revendications

1. Procédé d'alkylation d'une charge en hydrocarbure saturée qui comprend la mise en contact d'une charge en hydrocarbure devant être alkylée avec un agent d'alkylation en présence d'un catalyseur comprenant:
un acide solide, dans lequel ledit acide solide est une zéolithe choisie parmi le groupe composé de zéolithes X et zéolithes Y, y compris les zéolithes H-Y et les zéolithes USY;
un composant d'hydrogénation essentiellement composé d'un ou de plusieurs métaux nobles du groupe VIII, ledit composant d'hydrogénation étant présent en une quantité entre 0,01 à 2% poids calculé sous forme de métal et en fonction du poids total de la composition de catalyseur,
0,05 à 1% de soufre, en fonction du poids total de la composition de catalyseur et calculé sous forme de soufre, et
aucun composant halogène.

2. Procédé selon la revendication 1, dans lequel la charge en hydrocarbure comprend un isoalkane ayant de 4 à 10 atomes de carbone et l'agent d'alkylation comprend une oléfine ayant de 2 à 10 atomes de carbone.

3. Procédé d'alkylation d'un hydrocarbure qui comprend la mise en contact de la charge en hydrocarbure saturée devant être alkylée avec un agent d'alkylation en présence d'un catalyseur comprenant :
un acide solide, dans lequel ledit acide solide est une zéolithe choisie parmi le groupe composé de zéolithes X et zéolithes Y, y compris les zéolithes H-Y et les zéolithes USY;
un composant d'hydrogénation essentiellement composé d'un ou de plusieurs métaux nobles du groupe VIII, ledit composant d'hydrogénation étant présent en une quantité entre 0,01 à 2% poids calculé sous forme de métal et en fonction du poids total de la composition de catalyseur,
0,05 à 1% de soufre, en fonction du poids total de la composition de catalyseur et calculé sous forme de soufre, et
aucun composant halogène,
dans lequel la charge en hydrocarbure comprend un composé contenant du soufre.

4. Procédé selon la revendication 3, dans lequel l'acide solide comprend la zéolithe Y avec une taille de cellule unitaire de 24,34 à 24,72 angströms.

5. Procédé selon l'une quelconque des revendications 1 à 4, dans lequel le métal noble du groupe VIII est choisi parmi la platine, le palladium et des mélanges de ceux-ci.

6. Procédé selon l'une quelconque des procédés 1 à 5, dans lequel le composant d'hydrogénation comprend la platine.

7. Catalyseur qui peut être utilisé dans l'alkylation des hydrocarbures qui comprend:
une zéolithe choisie parmi le groupe composé de zéolithes X et zéolithes Y, y compris les zéolithes H-Y et les zéolithes USY;
un composant d'hydrogénation essentiellement composé d'un ou de plusieurs métaux nobles du groupe VIII mais comprenant toujours de la platine, ledit composant d'hydrogénation étant présent en une quantité entre 0,01 à 2% poids calculé sous forme de métal et en fonction du poids total de la composition de catalyseur,
0,05 à 1% de soufre, en fonction du poids total de la composition de catalyseur et calculé sous forme de soufre, et
aucun composant halogène.

8. Catalyseur selon la revendication 7, dans lequel la zéolithe comprend la zéolithe Y ayant une taille de cellule unitaire 24,34 à 24,72 angströms.

9. Procédé de préparation du catalyseur de la revendication 7 ou la revendication 8, lequel procédé comprenant l'étape de la mise en contact d'un matériau contenant la zéolithe et le composant d'hydrogénation avec un composant contenant du soufre.

10. Procédé selon la revendication 9, dans lequel le matériau contenant la zéolithe et le composant d'hydrogénation est calciné et ensuite réduit avant d'être mis en contact avec le composant contenant du soufre.

11. Procèdé selon la revendication 9 ou 10, dans lequel le matériau comprenant la zéolithe et le composant d'hydrogénation est réduit après avoir été mis en contact avec le composé contenant du soufre.
